(19) Europäisches Patentamt
European Patent Office
Office européen des brevets

(11) Veröffentlichungsnummer: **0 152 459**
**B1**

(12) # EUROPÄISCHE PATENTSCHRIFT

(45) Veröffentlichungstag der Patentschrift: **02.11.88**

(51) Int. Cl.⁴: **C 07 H 21/00**, C 07 H 19/04, C 07 F 9/26

(21) Anmeldenummer: **84903173.7**

(22) Anmeldetag: **10.08.84**

(86) Internationale Anmeldenummer: **PCT/EP 84/00244**

(87) Internationale Veröffentlichungsnummer: **WO 85/00816 (28.02.85 Gazette 85/05)**

(54) **VERFAHREN ZUR HERSTELLUNG VON OLIGONUCLEOTIDEN.**

(30) Priorität: **18.08.83 DE 3329892**

(43) Veröffentlichungstag der Anmeldung: **28.08.85 Patentblatt 85/35**

(45) Bekanntmachung des Hinweises auf die Patenterteilung: **02.11.88 Patentblatt 88/44**

(84) Benannte Vertragsstaaten: **AT BE CH DE FR GB LI LU NL SE**

(56) Entgegenhaltungen:
**EP-A-0 040 099**
**EP-A-0 061 746**
**EP-A-0 090 789**

**Journal of the American Chemical Society, Vol. 98, No. 12, 09 June 1976, R.L. Letsinger et al. 'Synthesis of thymidine oligonucleotides by phosphite triester intermediates' pages 3655-3661, see page 3656, Schema 1, page 3657; 2,2,2-Trichloroethyl Phosphotriesters - page 3658; cited in the application**
**Chemical Reviews, Vol. 77, No. 2, April 1977, V. Amarnath et al.: 'Chemical synthesis of oligonucleotides' pages 183-217, see pages 194-195**
**Tetrahedron Letters, Vol. 22, No. 20, 1981, S.L. Beaucage et al;'Deoxynucleoside phosphoramidites - A new class of key intermediates for deoxypolynucleotide synthesis:'**

(73) Patentinhaber: **BIOSYNTECH Biochemische Synthesetechnik GmbH, Stresemannstrasse 268-280, D-2000 Hamburg 50 (DE)**

(72) Erfinder: **SINHA, Nanda, Dual, Julius- Vosseler-Str. 32, D-2000 Hamburg 54 (DE)**
Erfinder: **Köster, Hubert, Prof. Dr., Hallerstr. 74, D-2000 Hamburg 13 (DE)**

(74) Vertreter: **Henkel, Feiler, Hänzel & Partner, Möhlstrasse 37, D-8000 München 80 (DE)**

(56) Entgegenhaltungen: (Fortsetzung)
**pages 1859-1862, see page 1859**
**Can. J. Chem. Vol. 58, 1980, p. 2686-2693**

**EP 0 152 459 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

**Beschreibung**

Die Erfindung betrifft ein Verfahren zur Herstellung von Oligonucleotiden der im Anspruch 1 angegebenen allgemeinen Formel I. Die erfindungsgemäß hergestellten Oligonucleotide weisen definierte Sequenzen auf und können als spezifische Primer und Probes eingesetzt werden bzw. sind für die Synthese kompletter Gene von großer Bedeutung (Arzneimittelforschung 30, 3a, 548, (1980)).

Oligonucleotide werden nach dem neusten Stand der Technik entweder nach der Phosphat- oder Phosphittriestermethode unter Verwendung polymerer Träger hergestellt (Nachr. Chem. Tech. Lab. 29, 230 (1981)). Um definierte Sequenzen aufbauen zu können, müssen die einzelnen Bausteine (Nucleoside bzw. Nucleotide) mit geeigneten Schutzgruppen versehen werden. Hier werden für den Schutz der exocyclischen Aminogruppen der heterocyclischen Nucleobasen allgemein basenlabile Acylgruppen, für die Verankerung der Oligonucleotidkette mit dem polymeren Träger in üblicher Weise eine basenlabile Esterbindung und für den Schutz der primären 5'-OH-Gruppe die säurelabilen Tritylethergruppen verwendet. Als Phosphatschutzgruppe der Phosphattriester-Methode wird üblicherweise entweder die 2-Chlorphenyl- oder 4-Chlorphenylgruppe in esterartiger Bindung verwendet, die nur durch den Angriff einer Base oder eines Nucleophils am Phosphoratom entfernt werden kann. Ein solcher Schritt ist an sich unerwünscht, da damit die Gefahr einer Spaltung der internucleotidischen Phosphatesterbindung gegeben ist. Diese Gefahr wurde durch die Verwendung von Oximat-Anionen (Tetrahedron Lett. 19, 2727 (1978)) stark reduziert, obwohl diese auch im entscheidenden Schritt in unerwünschter Weise am Phosphoratom angreifen und darüberhinaus den Nachteil aufweisen, daß eine verhältnismäßig geringe Menge an erwünschtem Oligonucleotid mit sehr großen Mengen an nicht flüchtigen und schwer extrahierbaren Salzen verunreinigt ist. Dies erschwert nicht nur die Aufarbeitung und nachfolgende Reinigung des synthetisierten Oligonucleotids, sondern führt auch zu erheblichen Substanzverlusten.

In der Phosphittriester-Methode wird üblicherweise die Methylgruppe in esterartiger Bindung als Phosphatschutzgruppe verwendet, die durch Angriff eines Nucleophils am Methyl-C-Atom entfernt werden kann (J. Amer. Chem. Soc. 99, 3526 (1977)). Da ein Angriff am P-Atom vermieden wird, wird die Gefahr einer Spaltung der Internucleotidbindung ebenfalls vermieden. Als Nucleophil wird üblicherweise Thiophenol/Triethylamin verwendet, die unangenehm zu handhaben sind und außerdem zu nicht flüchtigen, schwer extrahierbaren Verbindungen führen, die - wie oben erwähnt - sowohl die Aufarbeitung erschweren als auch zu erheblichen Materialverlusten führen.

Aus der EP-A-0 061 746 ist ein Verfahren zur Herstellung eines Oligonucleotids bekannt, bei dessen Herstellung u.a. die 3'-OH- oder 5'-OH-Gruppe eines gegebenenfalls an ein anorganisches Polymeres kovalent gebundenen Nucleosids oder Oligonucleotids mit einem Nucleosidphosphit der Formeln:

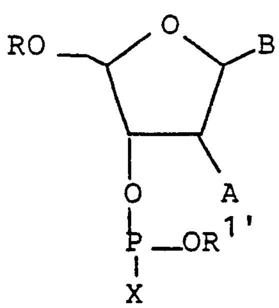

oder

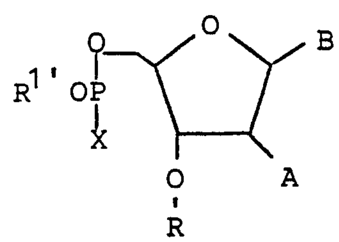

worin bedeuten:

B eine Nucleosid- oder Deoxynucleosidbase;

A H, OH oder $OR^4$, worin $R^4$ eine Blockierungsgruppe ist,

R eine Blockierungsgruppe,

$R^{1'}$ einen Kohlenwasserstoffrest mit bis zu 10 Kohlenstoffatomen und

X $NR^{2'}R^{3'}$, worin $R^{2'}$ und $R^{3'}$ einzeln jeweils Alkyl, Aryl, Aralkyl, Cycloalkyl und Cycloalkylalkyl mit bis zu 10 Kohlenstoffatomen bedeuten; $R^{2'}$ und $R^{3'}$ zusammen eine Alkylenkette, enthaltend bis zu 5 Kohlenstoffatome in der Hauptkette und insgesamt bis zu 10 Kohlenstoffatome bedeuten, wobei beide Endvalenzbindungen der genannten Kette an das Stickstoffatom, an welches $R^{2'}$ und $R^{3'}$ gebunden sind, gebunden sind und $R^{2'}$ und $R^{3'}$ zusammen mit dem Stickstoffatom, an welches sie gebunden sind, einen gesättigten Stickstoffheterocyclus bedeuten, der gewünschtenfalls wenigstens ein zusätzliches Heteroatom aus der Gruppe bestehend aus Stickstoff, Sauerstoff und Schwefel, enthält, bedeuten,

kondensiert wird. Dieses Verfahren geht davon aus, daß bei der Herstellung von sequenzdefinierten Oligonucleotiden die Verwendung trägergestützter Nucleosidzwischenprodukte bereits bekannt war (vgl. H. Köster in "Tetrahedron Letters", 1527-1530, 1972). Von W.B. Lunsford wird in "J. Amer.Chem.Soc." 98 : 12, 3655-3661 die Herstellung von Phosphotriesterderivaten von Oligothymidylaten beschrieben. Schließlich sind aus der EP-A-39 719 Verfahren zur Erzeugung von Internucleotidbindungen, d.h. von in einem Oligonucleotid oder Polynucleotid Nucleoside verknüpfenden Bindungen, durch Reaktion von Halophosphoriditen mit geeignet

blockierten Nucleosid- oder Oligonucleotidmolekülen bekannt.

Obwohl die eigentliche Synthese von Oligonucleotiden nach der Festphasen - Phosphit - bzw. Phophattriester-Methode recht effizient und schnell verläuft, ist die Herstellung von Oligonucleotiden definierter Sequenz nach wie vor sehr Zeitaufwendig. Dies liegt vor allem an den Problemen der nachfolgenden Aufarbeitung und Reinigung, die ein Mehrfaches der eigentlichen Synthesezeit beanspruchen. An diesem Punkt setzt das Verfahren der Erfindung ein, das hier eine entscheidende technische Verbesserung bietet.

Um zu Verbindungen der im Anspruch 1 angegebenen Formel I zu gelangen, in denen B eine Nucleobase, z. B. Adenin (A), Guanin (G), Cytosin (C), Thymin (T) oder Uracil (U) oder deren Analoga und $R^1$ Wasserstoff, Hydroxyl bzw. mit in der Nucleotidchemie üblichen Schutzgruppen geschütztes Hydroxyl sowie n eine ganze Zahl von 1 bis 200 bedeuten, sind erfindungsgemäß verschiedene, definierte Reaktionsschritte dürchzuführen:

a) Umsetzung eines Nucleosids der allgemeinen Formel II.

$R^1$ der allgemeinen Formel II kann Wasserstoff sein; es handelt sich dann bei den Verbindungen der Formel I um Oligodesoxynucleotide. Die Gruppe $R^1$ kann auch Hydoxyl- oder gegebenenfalls mit in der Nucleotidchemie üblichen Schutzgruppen geschütztes Hydroxyl sein. Derartige Schutzgruppen sind z. B. Trityl, Monomethoxytrityl und Dimethoxytrityl, Acyl, z. B. Acetyl, Benzoyl; Tetrahydropyranyl, Methoxytetrahydropyranyl, O-Nitrobenzyl sowie Silylether wie z. B. t-Butyl-Diphenylsilylether. Eine allgemeine Übersicht über in der Nucleotidchemie übliche Schutzgruppen findet sich z. B. in Tetrahedron 1981, Seite 363 - 369, Liebigs Ann. Ghem, 1978, 839 - 850, sowie Nucleic Acids Research, Symposium Series No. 7, 1980, 39 - 59.

$R^2$ ist ebenfalls eine in der Nucleotidchemie übliche Schutzgruppe gemäß den vorgenannten Veröffentlichungen, vorzugsweise die säurelabile 4,4-Dimethoxy- oder 4,4,4-Trimethoxytritylgruppe B' kann ebenfalls eine in der Nucleotidchemie übliche Schutzgruppe gemäß den oben genannten Vorveröffentlichungen aufweisen.

Das Nucleosid der Formel II wird erfindungsgemäß mit einem Phosphinderivat der allgemeinen Formel III gemäß Anspruch 1 umgesetzt.

In der allgemeinen Formel bedeutet X Chlor, Brom, CN oder SCN; L bedeutet CN, SCN oder einen Aminrest der Formel $-NR_2^4$ (Formel VIII) , wobei die Gruppen $R^4$ primäre, sekundäre oder tertiäre Alkyreste mit 1 - 10 Kohlenstoffstomen sind oder zusammen einen Cycloalkylrest mit 5 - 7 Kohlenstoffatomen, gegebenenfalls mit Alkylverzweigungen, und/oder einem oder zwei Stickstoff-, Sauerstoff- und/oder Schwefelatomen als Heteroatome enthalten kann, bedeuten. Die Gruppe L kann auch einen reaktiven heterocyclischen Rest bilden, der Imidazolyl, Triazolyl, Tetrazolyl, 3-Nitro-1,2,4-triazolyl, Thiazolyl, Pyrrolyl, Benztriazolyl oder Benzhydroxytriazolyl und dergleichen bildet.

$R^3$ des Phosphinderivats der allgemeinen Formel (III) ist erfindungsgemäß eine mit Hilfe von Basen durch 3-Eliminierung entfernbare Gruppe der allgemeinen Formel VII, in der Y Wasserstoff, Methyl oder Ethyl bedeutet. Z stellt eine elektronenziehende Gruppe dar, z. B. Halogen wie Fluor, Chlor oder Brom, CN, $NO_2$. Z kann weiterhin Phenyl, Phenylthio, Phenylsulfoxy oder Phenylsulfonyl bedeuten, wobei die Phenylreste in o,o' -Stellung und/oder p-Stellung mit Halogen, CN oder $NO_2$ substituiert sein können. Anstelle der Gruppe

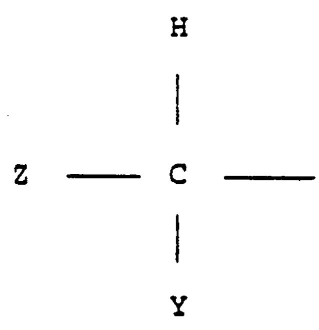

kann auch eine der Gruppen $CF_3$, $CCl_3$ oder $CBr_3$ stehen.

Die Umsetzung gemäß Schritt a erfolgt in Gegenwart einer organischen Base.

b) Umsetzung des in Schritt a erhaltenen Nucleosidphosphorigsäure-Derivates der Formel IV.

Die Umsetzung der Verbindung gemäß Formel IV erfolgt mit einem an einen polymeren Träger gebundenen Nucleosid der allgemeinen Formel V gemäß Anspruch 1. Es können lösliche oder unlösliche, d.h. vernetzte, polymere Träger verwendet werden, z. B. modifiziertes Silicagel, Glas, insbesondere "controlled pore glass", Polyester, Polyamid, Polyvinylalkohol, Polysiloxan, Polystyrol oder dergleichen. Als Verankerungsfunktion zwischen Träger und Nucleosid kommen vorzugsweise Esterbindungen in Betracht, einschließlich solcher, die sich von Lävulinyl- oder β-Benzoylpropionylrest ableiten; die letztgenannten Esterbindungen können unter neutralen Bedingungen mit Hydrazin gespalten werden. Auch die säurelabile Trityletherbindung,

gegebenenfalls mit Substiuenten in den Phenylringen, kommt als Verankerungsmöglichkeit in Betracht, vgl. Liebigs Ann. Chem. 1974, 959.

c) Oxidation des in Stufe b erhaltenen trägergebundenen Nucleotidonucleosids der allgemeinen Formel VI.

Die Oxidation führt zu einer Phosphatgruppe; sie kann z. B. mit Jod/$H_2O$, $H_2O_2$ oder organischen Persäuren oder allgemein durch Oxidation durch Einführung von O, S oder Se durchgeführt werden.

d) Maskierung freier primärer 5'-OH-Gruppen, die bei der Reaktion gemäß Stufe b (im Produkt der Formel V) nicht umgesetzt wurden.
Diese freien Hydroxylgruppen werden mit einer permanenten Schutzgruppe maskiert, z. B. durch Reaktion mit Acetanhydrid.

e) Abspaltung der Schutzgruppe(n) $R^2$

Die Abspaltung erfolgt beispielsweise unter Verwendung einer Protonsäure oder Lewissäure wie $ZnBr_2$ oder Dialkylaluminiumchlorid, wenn $R^2$ eine Tritylgruppe oder Methoxyderivat derselben darstellt.

f) Einführung weiterer Nucleosidphosphat- oder Oligonucleosidphosphateinheiten

Die Stufen a - e können wiederholt werden, wobei mindestens ein Nucleosidphosphatrest eingeführt wird. Beim Einsatz von Oligonucleosidphosphateinheiten werden selbstverständlich Kettenverlängerungen um mehr als eine Nucleosidphosphateinheit erreicht.

g) Abspaltung sämtlicher Schutzgruppen

Diese Abspaltung kann in der Weise erfolgen, daß mit wässrigem Ammoniak in einem Schritt die N-Acylgruppen der heterocyclischen Basen, die Esterbindung zwischen Oligonucleotid und Träger (gegebenenfalls kann letztere unter neutralen Bedingungen auch mit Hydrazin gespalten werden) und die Phosphatschutzgruppe gemäß dem allgemeinen Schema 1 am Schluß der Beschreibung durch β-Eliminierung abgespalten werden. Es wird dann ein Oligonucleotid mit nur 5'-terminaler Tritylschutzgruppe erhalten, das in an sich bekannter Weise nach Entfernung der flüchtigen Base (Ammoniak) direkt durch Hochdruckflüssigkeitschromatographie (HPLC) an "reversed phase"-Material gereinigt werden kann.

Die Zwischenprodukte der allgemeinen Formel IV stellen - abgesehen von den ausdrücklich ausgenommenen Verbindungen - neue Verbindungen dar. Sie liegen in Form recht stabiler, in reiner Form darstellbarer Verbindungen vor, sind leicht zu handhaben und dennoch recht reaktiv im Sinne der Knüpfung von Internucleotidbindungen. Der Einsatz von $R^3$ als eine durch Basen über β-Eliminierung entfernbare Schutzgruppe ermöglicht es zum ersten Mal, bis auf die 5'-Tritylgruppe alle Schutzgruppen in einem Schritt abzuspalten, wobei vorteilhafterweise durch Verwendung flüchtiger Basen das gewünschte Oligonucleotid in nur sehr geringem Maße mit Fremdstoffen verunreinigt ist und damit direkt nachfolgend durch die noch vorhandene hydrophobe 5'-Tritylgruppe über "reversed phase"-HPLC gereinigt werden kann.

Ein weiterer Vorteil des Verfahrens der Erfindung ergibt sich daraus, daß durch die Entfernung der Schutzgruppe durch β-Eliminierung kein Angriff am P-Atom erfolgt und damit an keiner der neu geknüpften Internucleotidbindungen während des Entschützens gespalten werden kann. Das Verfahren der Erfindung führt damit bei stark reduziertem Zeitaufwand zu insgesamt reineren Produkten als die bisher verfügbaren Verfahren.

Die Erfindung wird im folgenden anhand von Beispielen näher erläutert, wobei Phophinderivate zur Anwendung kommen, in denen $R^3$ ein β-Cyanethylgruppe ist. Einzelheiten der Reaktion und physiklische Kenndaten der hergestellten Verbindungen ergeben sich aus den Schemata 2 und 3, der Tabelle 1, sowie den Figuren 1 - 7 am Schluß der Beschreibung.

**Beispiel 1:**

Herstellung von Phosphinderivaten der allgemeinen Formel III:

Monochlor-β-cyanethyl-phosphoramidite:

Eine allgemeine Übersicht über die Reaktion ist aus Schema 1 ersichtlicht.

Dichlor-β-cyanethoxyphospin (1) wird mit einigen Verbesserungen im übrigen jedoch wie in Can. J Chem. 58, 2686 (1980)) dargestellt:

137,5 g (1,0 Mol) $PCl_3$ werden in einem Dreihalskolben mit Tropftrichter mit 300 ml Ether und 79,0 g (1 Mol) Pyridin versetzt; die Mischung wird auf -78° unter Argon abgekühlt. Dann wird eine Lösung von 71,0 g (1 Mol) β-Cyanethanol in 150 ml trockenem Ether tropfenweise über 1 bis 1,5 Stunden gegeben. Das Kältebad wird

entfernt; man rührt weitere 3 Stunden bei Raumtemperatur (unter Umständen werden erneut 300 ml Ether zugegeben, um eine bessere Rührfähigkeit zu gewährleisten). Rührer und Tropftrichter werden unter Argon entfernt; das Gemisch wird über Nacht bei 0°C aufbewahrt. Die festen Salze werden unter Argon entfernt; der Niederschlag wird zweimal mit je 75 ml Ether gewaschen. Die vereinigten organischen Phasen werden im Vakuum konzentriert; der Rückstand wird schließlich im Vakuum destilliert: Siedepunkt 70 - 75°C/53,2 Pa (70 - 75°C/0,4 mm).

Monochlor-β-cyanethylphosphoramidite (3):

Zu einer Lösung des N-trimethylsilylierten sekundären Amins (0,1 Mol) oder sekundären Amin (0,2 Mol) in 30 ml Ether wird tropfenweise bei -20°C unter Argon eine Lösung von 17,2 g (0,1 Mol) β-Cyanethylphosphordichloridit (1) in 60 ml Ether im Verlauf von 1 bis 1,5 Stunden zugetropft. Nach 20stündigem Rühren bei Raumtemperatur wird das Aminhydrochlorid entfernt; die verbleibende Lösung wird konzentriert. Der Rückstand wird schließlich im Vakuum in einer Kurzwegdestille destilliert.

Die physikalischen Eigenschaften der so erhältlichen Verbindungen sind in Tabelle 1 zusammengefasst.

Die Figuren 1a, 1b und 1c zeigen $^{31}P$-NMR-Spektren von drei verschiedenen Monochlor-β-cyanethylphosphoramiditen.

Das N-Morpholinderivat ist thermisch zu instabil, um destilliert werden zu können. Die Präparation ist dennoch so rein, daß der Rückstand direkt für die Herstellung der aktivierten Nucleosid-Derivate verwendet werden kann. Die Reinheit ist gewöhnlich entsprechend den $^{31}P$-NMR-Spektren größer als 95 %.

Nucleosid-β-cyanethylphosphoramidite:

Die Darstellung der entsprechend geschützten Nucleosid-β-cyanethylphosphoramidite ist aus Schema 3 ersichtlich.

Die Synthese gelingt in Analogie zu Tetrahedron Lett. 22, 1859 (1981) mit einigen Verbesserungen in guten Ausbeuten.

3,0 m Mol des N-geschützten 5'-dimethoxytritylierten Desoxynucleosids werden mit THF/Toluol azeotrop getrocknet, in 15 ml trocknen THF gelöst und mit 12,0 m Mol N, N, N-Diisopropylethylamin zugegeben. Zu dieser Lösung werden unter kräftigem Rühren tropfenweise im Verlauf von 2 Minuten unter Argon 6,0 m Mol des Monochlor-β-cyanethylphosphoramidits zugefügt. Nach kurzer Zeit (2 bis 5 Minuten) fällt Aminhydrochlorid aus. Die Suspension wird für weitere 30 bis 40 Minuten gerührt. Aminhydrochlorid wird unter Argon abfiltriert und gründlich mit trockenem THF (10 bis 15 ml) nachgewaschen. Die gesamte organische Phase wird konzentriert und in Argon-gesättigtem Ethylacetat gelöst (100 ml). Die organische Phase wird zweimal mit je 50 ml Argon-gesättigter 10 %-wässriger Natriumcarbonatlösung extrahiert. Die organischen Phasen werden mit Natriumsulfat getrocknet und unter reduziertem Druck zu einem Schaum eingedampft. Der Schaum wird mit wenig Ethylacetat oder Toluol aufgelöst und in n-Hexan bei -78°C gefällt. Die aktivierten Nucleoside sind stabil für mehrere Monate, wenn man sie unter Argon bei -20°C aufbewahrt.

Figur 2 zeigt das $^{31}P$-NMR-Spektrum eines der aktivierten Desoxynucleoside.

Synthese von d (CGGTACCG)

100 mg "controlled pore glass" (CPG) , beladen mit insgesamt 8 μ Mol N-Isobutyryl-desoxyguanin (vgl. Tetrahedron Lett. 24, 747 (1983)) wird nacheinander mit den 5'-dimethoxytritylierten N-acylierten β-Cyanethyl-N,N-diisopropylphosphoramiditen der Desoxynucleoside C, C, A, T, G, G und C kondensiert, wobei jeweils 20 bis 25 Äquivalente des Phosphoramidites in Acetonitril mit jeweils 75 - 80 Äquivalenten sublimiertem Tetrazol aktiviert werden. Die Kondensationen sind nach längstens 30 Minuten beendet; die Kopplungsausbeute beträgt mehr als 94 %. Nach der Kondensation folgt jeweils eine Oxidation mit $J_2/H_2O$ und Maskierung nicht umgesetzter 5'-OH-Gruppen mit Acetanhydrid. Anschließend erfolgt Abspaltung der Dimethoxytritylgruppe entweder mit 3 % Trichloressigsäure in Nitromethan/1 % Methanol oder $ZnBr_2$/Nitromethan/1 % $H_2O$.

Die Gesamtausbeute des geschützten Oktanucleotids am Ende aller Kondensationsschritte beträgt 55 %, bezogen auf das trägergebundene Desoxyguanosin.

Die vollständige Entschützung und Abspaltung vom Träger wird in einem Schritt durch Umsetzung der Glasperlen mit konzentriertem wässrigen Ammoniak (3 ml) bei 50°C in 16 Stunden erreicht. Anschließend werden die Glasperlen gründlich mit 50 % wässrigem Methanol gewaschen (3 mal mit je 3 ml) Die flüssige Phase wird durch Einengen (Entfernung des Methanols) und Gefriertrocknung entfernt. Anschließend wird ein Aliquot nach Filtration durch Millipore-Filter durch HPLC an RP 18 gereinigt, wie aus Figur 3 ersichtlich ist. Das reine Produkt eluiert in einem Peak bei einer Retentionszeit von etwa 13 min.

Die Fraktionen, die das 5'-dimethoxytritylierte Oligonucleotid enthalten, werden gesammelt; der flüchtige Puffer wird im Vakuum am Rotationsverdampfer entfernt. Der Rückstand wird mit 1 ml 80 %-iger Essigsäure versetzt. Die Essigsäure wird nach 45 Minuten bei Raumtemperatur durch Gefriertrocknung entfernt.

Das so erhaltene Material wird in üblicher Weise, (Liebigs Ann. Chem. 1978, 982) mit T4-Polynucleotidkinase und $\gamma^{32}P$-ATP phosphoryliert. Das erhaltene Produkt wird durch Polyacrylamidgelelektrophorese im Vergleich zu einem Homo-oligo-dT-Wellenlängenstandard (Nucleic Acids Res. 6, 2096 (1979) (Figur 4) und gemäß Figur 5 durch Sequenzierung (Liebigs Ann. Chem 1978, 982) charakterisiert.

Die Figuren 6a bis 6c zeigen die Ergebnisse (HPLC, Gelelektrophorese, Sequenzierung) der Synthese von d

(GGGATCCC) unter Verwendung der Nucleosid-β-cyanethyl-N,N-dimethylphosphoamidite. Die Figuren 7a bis 7c zeigen die Ergebnisse (HPLC, Gelelektrophorese, Sequenzierung der Synthese von d (GGGATATCCC) unter Verwendung der Nucleosid-β-cyanethyl-N-morpholinophosphoamidite. In den Fig. 6a und 7a eluiert das jeweilige Produkt in einem Peak bei einer Retentionszeit von etwa 12 min.

Die in den Figuren 3, 6a und 7a wiedergegebenen Ergebnisse wurden durch Anwendung eines Gradienten von 10-25 Vol-% $CH_3CN$, 5 min., und 25-29 Vol.-% $CH_3CN$, 30 min. in 0,1 M Triethylammoniumacetat bei pH 7,0 erhalten.

**Tabelle 1**: Physikalische Daten von Monochlor-β-cyanethylphophoramiditen

| Verbindung | 3a<br>L = N,N-Dimethylamino | 3b<br>L = N,N-Diisopropylamino | 3c[1]<br>L = N-Morpholino |
|---|---|---|---|
| Siedepunkt | 90-92°/0.6 mm | 103-5°/0.08 mm | |
| Chemischer Shift[2] für $^{31}P$-NMR in $CH_3CN$ | 175,97 ppm | 179,82 ppm | 168,22 ppm |
| Chemischer Shift für $^{1}H$-NMR in ppm | 4.01, 4.17 (2t, P-$OCH_2$,2H)<br>2.71 (t,-$CH_2$-CN,2H)<br><br>2.7 (d,N$(CH_3)_2$,6H) | 4.02, 4.2 (2t, $POCH_2$-2H)<br>3.8 (m,N$(CH)_2$,2H)<br>2.77 (t,-$CH_2$CN,2H)<br>1.29 (d,N-CH$(CH_3)_2$,12H) | 3.96, 4.1 (2t, $POCH_2$,2H<br>3.67 (t,O$(CH_2)_2$,4H)<br>3.17 (m,P-N$(CH_2)_2$,4H)<br>2.74 (t,$CH_2$-$CN_2$,2H) |
| Massen-spektrum | $(\frac{m}{e})^+$ = 180, 182 (+2), 145 (-Cl), 136 (-$C_2H_6N$), 110 (-$C_3N_4NO$) | $(\frac{m}{e})^+$ = 236, 238 (+2), 201 (-Cl), 166 (-$C_3H_4NO$), 136 (-$C_6H_{14}N$) | $(\frac{m}{e})^+$ = 222, 224 (+2), 187 (-Cl), 152 (-$C_3H_4NO$), 136 (-$C_4H_8O$) |

[1] Das Rohprodukt weist nach Abtrennung von Aminhydrochlorid und von im Hochvakuum bei Raumtemperatur flüchtigen Verbindungen nach dem $^{31}P$-NMR-Spektrum eine Reinhheit von 93-95 % auf.

[2] Die chemischen Shifts sind in Aceton-$d_6$ mit 80 %-iger $H_3PO_4$ als externem Standard gemeßen.

**Schema 1.**

Entfernung der Gruppe $R^3$ durch β-Eliminierung.

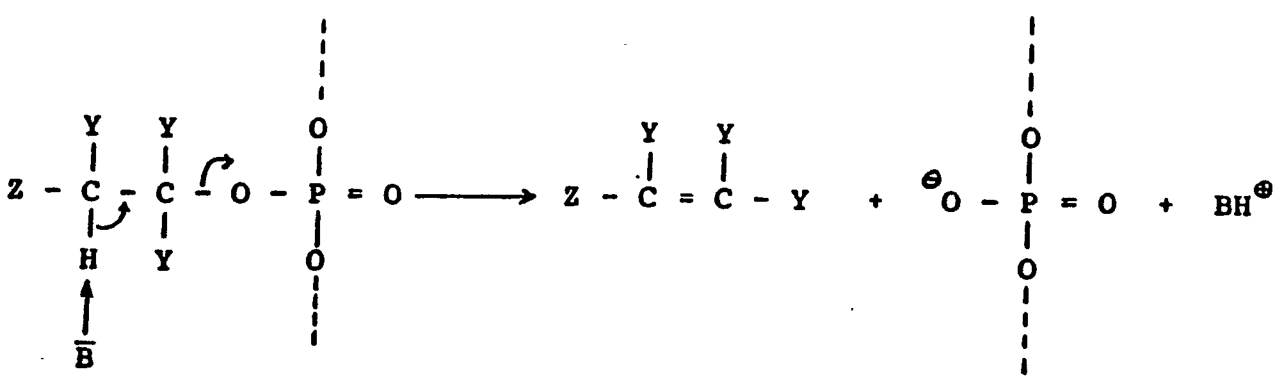

**Schema 2.**

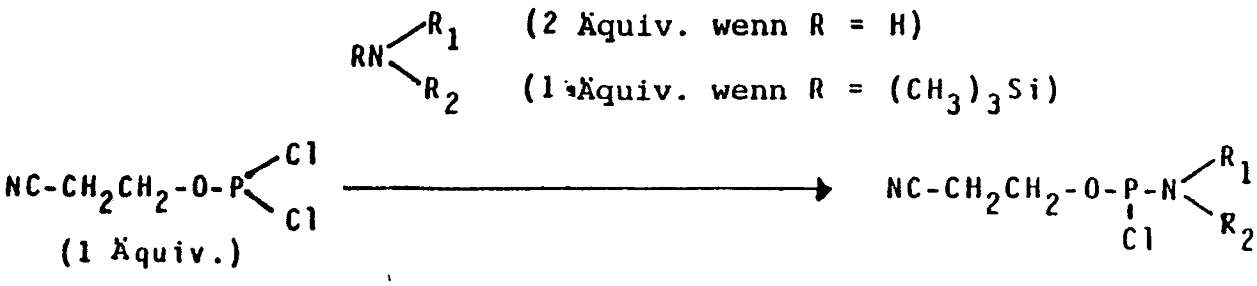


1 3

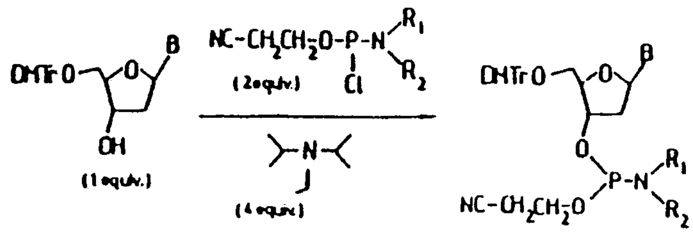

a) $R_1 = R_2 = CH_3$ b) $R_1 = R_2 =$ 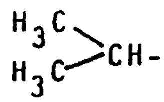

= Thymin , 2-(Methyl)benzoylcytosin

c) $R_1 + R_2$ = Morpholino

, Isobutyrylguanin , Benzoyladenin

## Patentansprüche

1. Verfahren zur Herstellung von Oligonucleotiden der allgemeinen Formel I

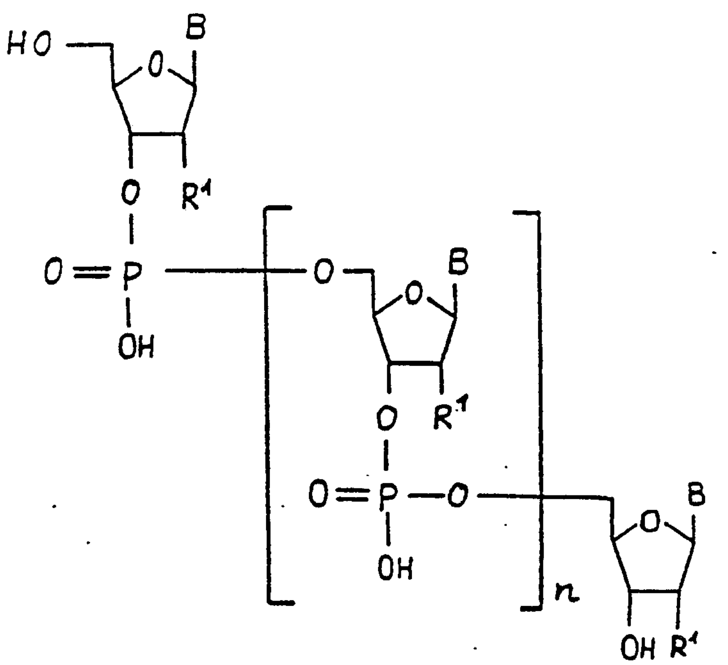

(I)

in der

B eine Nucleobase,

$R^1$ Wasserstoff, Hydroxyl oder mit in der Nucleotidchemie üblichen Schutzgruppen geschütztes Hydroxyl

und
n eine ganze Zahl von 1 - 200 bedeuten,
mit den folgenden Reaktionsschritten:

a) Umsetzung eines Nucleosids der allgemeinen Formel II

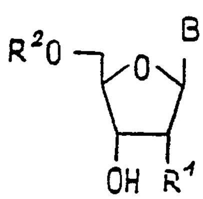

(II)

in der
$R^1$ wie oben definiert ist, sowie
$R^2$ eine in der Nucleotidchemie übliche Schutzgruppe und
B' die gegebenenfalls mit in der Nucleotidchemie üblichen Schutzgruppen geschützte Nucleobase B bedeuten,
mit einem Phosphinderivat der Formel III

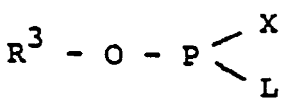

(III)

in der $R^3$ eine abspaltbare Schutzgruppe sowie X und L mit Hydroxylgruppen der Zuckerreste der Nucleotide bzw. Nucleoside reagiererde Gruppen sind, in Gegenwart einer Base,

b) Umsetzung des in Schritt a) erhaltenen Nucleotidderivats der Formel IV

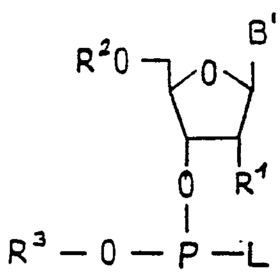

(IV)

in der B', $R^1$, $R^2$, $R^3$ und L wie oben definiert sind, mit einem an einen polymeren Träger gebundenen Nucleosid der allgemeinen Formel V

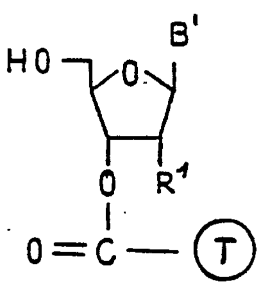

(V)

in der B' und $R^1$ wie oben definiert sind und T den polymeren Träger bedeutet,

c) Oxidation des in Schritt b) erhaltenen trägergebundenen Nucleosidonucleotids der allgemeinen Formel VI

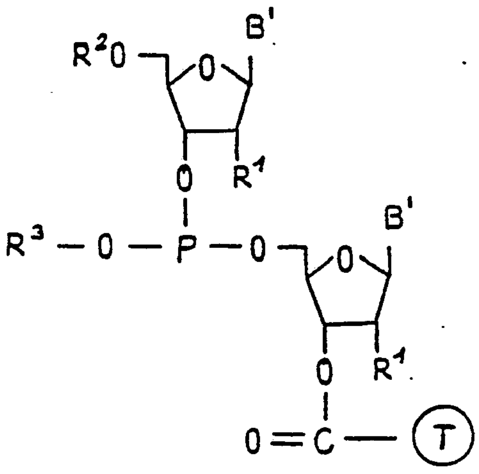

(VI)

in der B', $R^1$, $R^2$, $R^3$ und T wie oben definiert sind, unter Ausbildung von Phosphotriestergruppen.

d) Maskierung freier primärer 5'-OH-Gruppen, die bei der Reaktion gemäß Schritt b) nicht umgesetzt wurden, mit in der Nucleotidchemie üblichen, permanenten Schutzgruppen,

e) Abspaltung der Schutzgruppe $R^2$,

f) gegebenenfalls ein- oder mehrfache Wiederholung der Schritte a) bis e) zur Einführung weiterer Nucleosidphosphat- oder Oligonucleosidphosphateinheiten, sowie

g) Spaltung der Nucleosid-Trägerbindung und gegebenenfalls Abspaltung aller in den Oligonucleosidphosphaten vorhandenen Schutzgruppen,
dadurch gekennzeichnet, daß
man in Stufe a) als Phosphinderivat der allgemeinen Formel III eine Verbindung verwendet, in der $R^3$ eine Gruppe der Formel VII

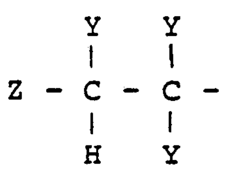

(VII)

in der bedeuten:
die Gruppen Y, die gleich oder verschieden sein können, Wasserstoff, Methyl und/oder Ethyl und
Z Halogen, CN, $NO_2$ oder gegebenenfalls in o,o'-Stellung und/oder p-Stellung halogen-, CN- oder $NO_2$-substituiertes Phenyl, Phenylthio, Phenylsulfoxy oder Phenylsulfonyl und in der

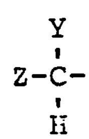

durch $CCl_3$-, $CF_3$- oder $CBr_3$- ersetzt sein kann;
wobei in dem Phosphinderivat der Formel III
X für Chlor, Brom, CN oder SCN steht, und
L CN oder SCN, einen sekundären Aminrest der Formel (VIII)

$-NR_2^4$ (VIII)

worin die Gruppen $R^4$ primäre, sekundäre oder tertiäre Alkylreste mit 1 - 10 Kohlenstoffatomen sind, oder zusammen einen gegebenenfalls alkylverzweigten Cycloalkylrest mit 5 - 7 Kohlenstoffatomen, der ein oder zwei Stickstoff-, Sauerstoff- und/oder Schwefelatome als Heteroatome enthalten kann, darstellen, oder einen Imidazolyl-, Triazolyl-, Tetrazolyl-, 3-Nitro-1,2,4-triazolyl-, Thiazolyl-, Pyrrolyl-, Benztriazolyl- oder Benzhydroxytriazolylrest,
bedeutet.

2. Verfahren nach Anspruch 1, dadurch gekennzeichnet, daß man ein Phosphinderivat der Formel (III)

einsetzt, in der X Chlor, L eine N,N-Dimethyl-, Diethyl- oder Diisopropylamingruppe oder N-Morpholinogruppe und $R^3$ eine β-Cyanethylgruppe ist.

3. Geschütztes Nucleotid der Formel:

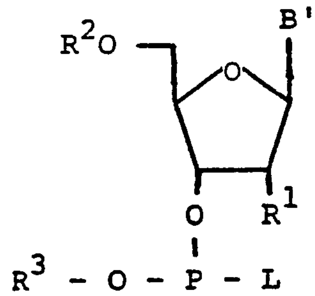

(IV)

worin bedeuten:

$R^1$ Wasserstoff, Hydroxyl oder mit in der Nucleotidchemie üblichen Schutzgruppen geschütztes Hydroxyl;

$R^2$ eine in der Nucleotidchemie übliche Schutzgruppe;

$R^3$ eine Gruppe der Formel:

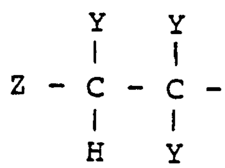

in der bedeuten:

die Gruppen Y, die gleich oder verschieden sein können, Wasserstoff, Methyl und/oder Ethyl und

Z Halogen, CN, $NO_2$ oder gegebenenfalls in o,o'-Stellung und/oder p-Stellung halogen, CN- oder $NO_2$-substituiertes Phenyl, Phenylthio, Phenylsulfoxy oder Phenylsulfonyl mit Ausnahme des Falles, daß sämtliche Gruppen Y = H und L = $-NR_2^4$ mit beiden Gruppen $R^4$ gleich Alkyl oder beiden Gruppen zusammen mit dem H-Atom gleich einem heterozyklischen Rest un in der

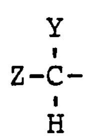

durch $CCl_3$-, $CF_3$- oder $CBr_3$- ersetzt sein kann; und

L CN oder SCN, einen sekundären Aminrest der Formel (VIII)

$-NR_2^4$ (VIII)

worin die Gruppen $R^4$ primäre, sekundäre oder tertiäre Alkylreste mit 1 - 10 Kohlenstoffatomen sind, oder zusammen einen gegebenenfalls alkylverzweigten Cycloalkylrest mit 5 - 7 Kohlenstoffatomen, der ein oder zwei Stickstoff-, Sauerstoff- und/oder Schwefelatome als Heteroatome enthalten kann, darstellen, oder einen Imidazolyl-, Triazolyl-, Tetrazolyl-, 3-Nitro-1,2,4-triazolyl-, Thiazolyl-, Pyrrolyl-, Benztriazolyl- oder Benzhydroxytriazolylrest, und

B' eine gegebenenfalls mit in der Nucleotidchemie üblichen Schutzgruppen geschützte Nucleobase.

4. Geschütztes Nucleotid nach Anspruch 3, dadurch gekennzeichnet, daß Z für -CN steht.

5. Geschütztes Nucleotid nach Anspruch 4, dadurch gekennzeichnet, daß $R^3$ für $-CH_2-CH_2-CN$ steht.

6. Geschütztes Nucleotid nach Anspruch 3, dadurch gekennzeichnet, daß $R^2$ für die 4,4'-Dimethoxy- oder 4,4',4''-Trimethoxytritylgruppe steht.

7. Geschütztes-Nucleotid der Formel:

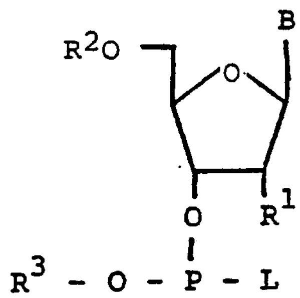

(IV)

worin bedeuten:

$R^1$ Wasserstoff, Hydroxyl oder mit in der Nucleotidchemie üblichen Schutzgruppen geschütztes Hydroxyl;
$R^2$ für die 4,4'-Dimethoxy- oder 4,4',4''-Trimethoxytritylgruppe steht;
$R^3$ $-CH_2-CH_2-CN$;
B' eine gegebenenfalls mit in der Nucleotidchemie üblichen Schutzgruppen geschützte Nucleobase und
L N,N-Dimethylamino, N,N-Diisopropylamino oder N-Morpholino.

## Claims

1. A process for the preparation of oligonucleotides of the general formula I

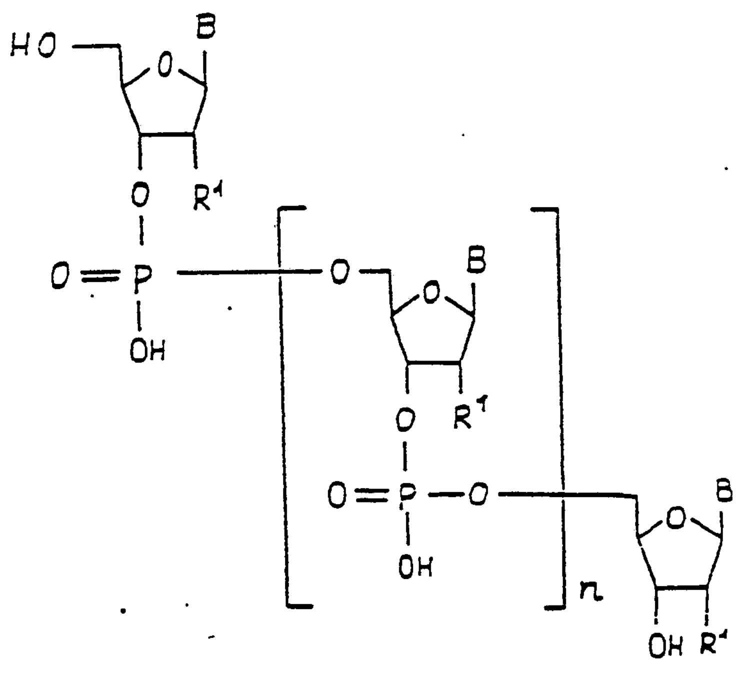

(I)

in which B denotes a nucleobase, $R^1$ denotes hydrogen, hydroxyl or hydroxyl which is protected by the protective groups customary in nucleotide chemistry, and n denotes an integer from 1 to 200, by the following reaction steps:

a) reaction of a nucleoside of the general formula II

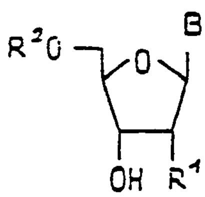

(II)

in which $R^1$ is defined as above, and $R^2$ denotes a protective group customary in nucleotide chemistry and B' denotes the nucleobase B protected, where appropriate, by the protective groups customary in nucleotide chemistry, with a phosphine derivative of the general formula III

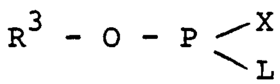

(III)

in which $R^3$ is a protective group which can be eliminated, and X and L are groups which react with hydroxyl groups in the sugar moieties of the nucleotides or nucleosides, in the presence of a base,

b) reaction of the nucleotide derivative of the formula IV obtained in step a)

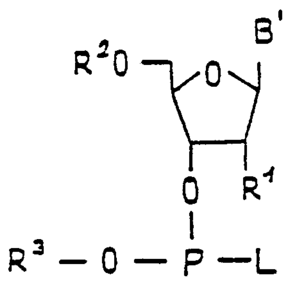

(IV)

in which B', $R^1$, $R^2$, $R^3$ and L are as defined above, with a nucleoside, of the general formula V, bound to a polymeric carrier

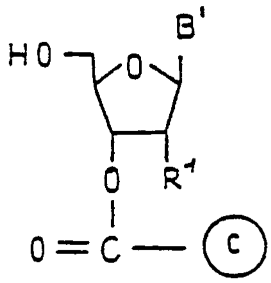

(V)

in which B' and $R^1$ are as defined above and C denotes the polymeric carrier :

c) oxidation of the carrier-bound nucleoside- nucleotide of the general formula VI, obtained in step b)

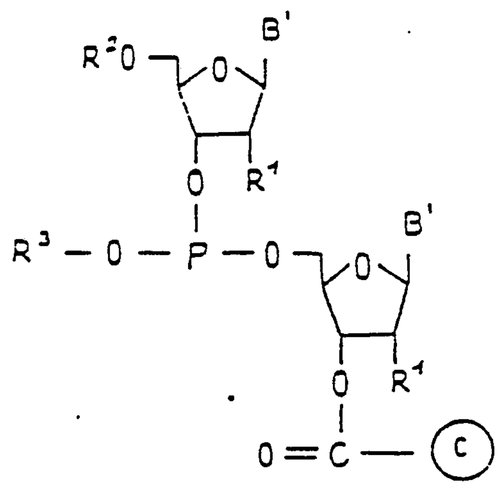

(VI)

in which B', $R^1$, $R^2$, $R^3$ and C are as defined above, with formation of phosphotriester groups,

d) blocking of free primary 5'-OH groups, which have not been reacted in the reaction according to step b), with permanent protective groups customary in nucleotide chemistry,

e) elimination of the protective group $R^2$,

f) where appropriate, single or multiple repetition of steps a) to e) to introduce further nucleoside phosphate or oligonucleoside phosphate units, and

g) cleavage of the nucleoside-carrier bond and, where appropriate, elimination of all protective groups present in the oligonucleoside phosphates,
which process comprises using in step a) as the phosphine derivative of the general formula III a compound in which $R^3$ denotes a group of the formula VII

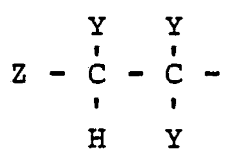

(VII)

in which the groups Y, which can be identical or different, represent hydrogen, methyl and/or ethyl and Z represents CN, $NO_2$ or optionally phenyl, phenylthio, phenylsulfoxy or phenylsulfonyl halogen, CN or $NO_2$-substituted in the o,o'-position and/or p-position and in which

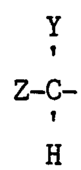

can be replaced by $CCl_3$, $CF_3$ or $CBr_3$ and in the phosphine derivative of Formula III X stands for chlorine, bromine, CN or SCN and
L is CN or SCN, a secondary amino radical of Formula (VIII)

$-NR_2^4$ (VIII)

in which the groups $R^4$ are primary, secondary or tertiary alkyl radicals with 1 to 10 carbon atoms, or together an optionally alklybranched cycloalkyl radical with 5 to 7 carbon atoms, which can contain one or two nitrogen, oxygen and/or sulphur atoms as heteroatoms, or an imidazolyl, triazolyl, tetrazolyl, 3-nitro-1,2,4-triazolyl, thiazolyl, pyrrolyl, benztriazolyl or benzhydroxytriazolyl radical.

2. Process according to claim 1, characterized in that use is made of a phosphine derivative of Formula (III), in which X is chlorine, L a N,N-dimethyl, diethyl or diisopropyl amine group or N-morpholino group and $R^3$ is a β-cyanethyl group.

3. Protected nucleotide of Formula:

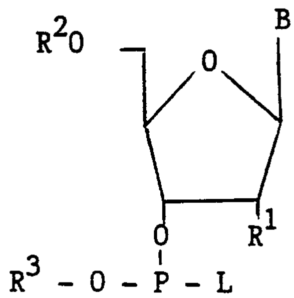

(IV)

in which:

$R^1$ stands for hydrogen, hydroxyl or hydroxyl protected by protective groups conventionally used in nucleotide chemistry;

$R^2$ a protective group conventionally used in nucleotide chemistry;

$R^3$ a group of formula:

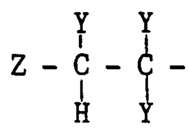

in which:

the groups Y, which can be the same or different, are hydrogen, methyl and/or ethyl and

Z is halogen, CN, $HO_2$ or optionally phenyl, phenylthio, phenylsulfoxy or phenylsulfonyl halogen, CNCH or $NO_2$-substituted in the o,o'-position and/or p-position, with the exception of the case that all the groups Y = H and L = $NR_2^4$ with both groups $R^4$ are alkyl or both groups together with the N-atom are a heterocyclic radical and can be replaced in the

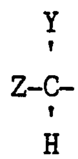

by $CCl_3$, $CF_3$ or $CBr_3$ and

L stands for CN or SCN, a secondary amino radical of formula (VIII)

$-NR_2^4$ (VIII)

in which the groups $R^4$ are primary, secondary or tertiary alkyl radicals with 1 to 10 carbon atoms, or together represent an optionally alkyl-branched cycloalkyl radical with 5 to 7 carbon atoms, which can contain one or two nitrogen, oxygen and/or sulphur atoms as heteroatoms, or an imidazolyl, triazolyl, tetrazolyl, 3-nitro-1,2,4-triazolyl, thiazolyl, pyrrolyl, benztriazolyl or benzhydroxytriazolyl radical and

B' is a nucleobase optionally protected with the protective groups conventionally used in nucleotide chemistry.

4. Protected nucleotide according to claim 3, characterized in that Z stands for -CN.

5. Protected nucleotide according to claim 4, characterized in that $R^3$ stands for $-CH_2-CH_2-CN$.

6. Protected nucleotide according to claim 3, characterized in that $R^2$ stands for the 4,4'-dimethoxy or 4,4',4''-trimethoxytrityl group.

7. Protected nucleotide of formula:

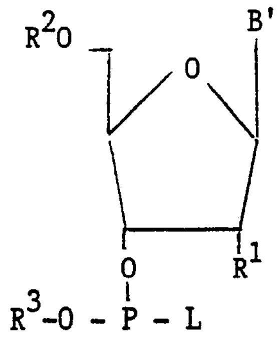

(IV)

in which:

$R^1$ stands for hydrogen, hydroxyl or hydroxyl protected with protective groups conventionally used in nucleotide chemistry;

$R^2$ stands for 4,4'-dimethoxy or 4,4',4''-trimethoxytrityl group;

$R^3$ stands for $-CH_2-CH_2-CN$;

B' stands for a nucleobase optionally protected by protective groups conventionally used in nucleotide chemistry and

L stands for N,N,-dimethylamino, N,N-diisopropylamino or N-morpholino.

**Revendications**

1. Procédé pour la préparation d'oligonucléotides de formule générale I

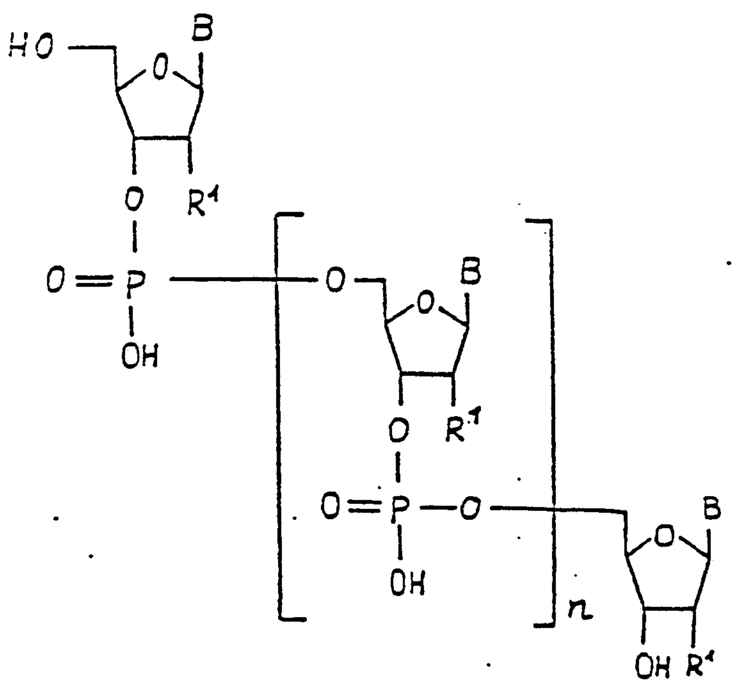

(I)

dans laquelle

B représente une base nucléotidique;

$R^1$ représente un atome d'hydrogène ou un groupe hydroxyle ou hydroxyle protégé par des groupements protecteurs usuels dans la chimie des nucléotides; et

n est un nombre entier valant de 1 à 200;

comportant les étapes de réaction suivantes:

a) réaction d'un nucléoside de formule générale II

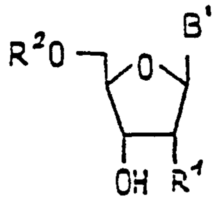

(II)

dans laquelle

$R^1$ est tel que défini plus haut,

$R^2$ représente un groupe protecteur usuel dans la chimie des nucléotides, et

B′ représente la base nucléotidique B éventuellement protégée par des groupes protecteurs usuels dans la chimie des nucléotides;

avec un dérivé de phosphine de formule générale III

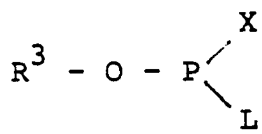

(III)

dans laquelle $R^3$ est un groupe protecteur séparable, et X et L sont des groupes réagissant avec les groupes hydroxyle des restes glucidiques des nucléotides ou, respectivement, nucléosides, en présence d'une base;

b) réaction du dérivé nucléotidique de formule IV

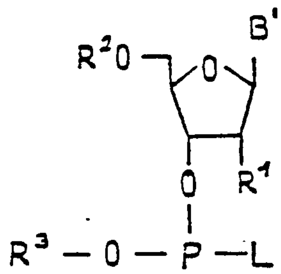

(IV)

obtenu dans l'étape a), dans laquelle formule B′, $R^1$, $R^2$, $R^3$ et L sont tels que définis plus haut, avec un nucléoside lié à un support polymère, de formule générale V

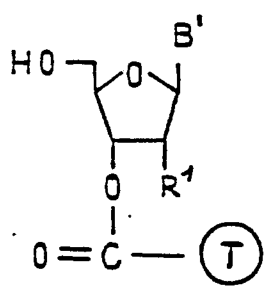

(V)

dans laquelle B′ et $R^1$ sont tels que définis plus haut et T représente le support polymère,

c) oxydation du nucléosidonucléotide de formule générale VI

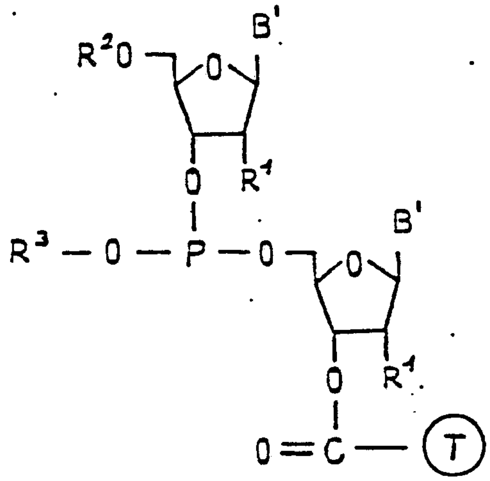

(VI)

lié à un support, obtenu dans l'étape b), dans laquelle formule B', $R^1$, $R^2$, $R^3$ et T sont tels que définis plus haut, avec formation de groupes phosphotriester;

d) blocage de groupes 5'-OH primaires libres, qui n'ont pas réagi lors de la réaction selon l'étape b), par des groupements protecteurs permanents, usuels dans la chimie des nucléotides;

e) élimination du groupe protecteur $R^2$;

f) éventuellement répétition une ou plusieurs fois des étapes a) à e) pour l'introduction de nouvelles unités nucléosidephosphate ou oligonucléosidephosphate; et

g) coupure de la liaison support-nucléoside et éventuellement élimination de tous les groupes protecteurs présents dans les oligonucléoside-phosphates;

caractérisé en ce que, dans l'étape a), on utilise, en tant que dérivé de phosphine de formule générale III, un composé dans lequel $R^3$ represente un groupe de formule VII

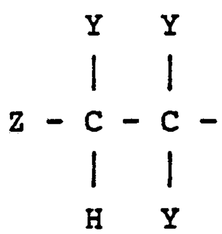

(VII)

dans laquelle

les groupes Y, qui peuvent être identiques ou différents, représentent des atomes d'hydrogène ou des groupes méthyle et/ou éthyle; et

Z représente un atome d'halogène, ou un groupe CN ou $NO_2$, ou un radical phényle, phénylthio, phénylsulfoxy ou phénylsulfonyle éventuellement substitué en position ortho,ortho' et/ou en position para par un atome d'halogène ou par CN ou $NO_2$, et dans laquelle

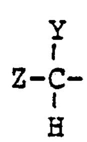

peut être remplacé par $CCl_3$-, $CF_3$- ou $CBr_3$-;

dans le dérivé de phosphine de formule III, X représentant un atome de chlore, de brome, CN ou SCN, et L représentant CN ou SCN, un reste amino secondaire de formule VIII

$$-NR^4_2 \qquad (VIII)$$

dans laquelle les groupes $R^4$ sont des restes alkyle primaires, secondaires ou tertiaires ayant de 1 à 10 atomes de carbone, ou forment ensemble un reste cycloalkyle ayant de 5 à 7 atomes de carbone, portant éventuellement des ramifications alkyle, qui peut contenir un ou deux atomes d'azote, d'oxygène et/ou de soufre en tant qu'hétéroatomes, ou un reste imidazolyle, triazolyle, tétrazolyle, 3-nitro-1,2,4-triazolyle,

thiazolyle, pyrrolyle, benzotriazolyle ou benzhydroxytriazolyle.

2. Procédé selon la revendication 1, caractérisé par le fait qu'on utilise un dérivé de phosphine de formule (III) dans lequel X est un atome de chlore, L est un groupe N,N-diméthyl-, diéthyl- ou diisopropylamino, ou le groupe N-morpholino, et $R^3$ est un groupe β-cyanoéthyle.

3. Nucléotide protégé de formule

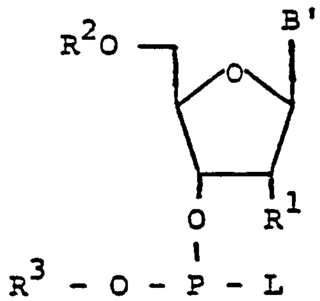

(IV)

dans laquelle

$R^1$ représente un atome d'hydrogène ou un groupe hydroxyle ou hydroxyle protégé par des groupements protecteurs usuels dans la chimie des nucléotides;

$R^2$ représente un groupe protecteur usuel dans la chimie des nucléotides;

$R^3$ représente un groupe de formule

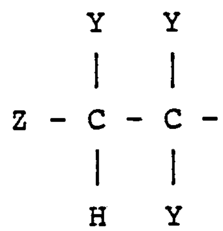

dans laquelle les groupes Y, qui peuvent être identiques ou différents, représentent un atome d'hydrogène ou un groupe méthyle et/ou éthyle, et Z représente un atome d'halogène ou un groupe CN, $NO_2$ ou un radical phényle, phénylthio, phénylsulfoxy ou phénylsulfonyle éventuellement substitué en position ortho,ortho' et/ou para par un atome d'halogène, CN ou $NO_2$, à l'exception du cas où tous les groupes Y = H et L = $-NR_2^4$, avec les deux groupes $R^4$ représentant un groupe alkyle ou les deux groupes formant avec l'atome d'azote un reste hétérocyclique; et dans laquelle

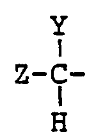

peut être remplacé par $CCl_3$-, $CF_3$- ou $CBr_3$-, et

L représente CN ou SCN, un reste amino secondaire de formule (VIII)

$-NR_2^4$ (VIII)

dans laquelle les groupes $R^4$ sont des restes alkyle primaires, secondaires ou tertiaires ayant de 1 à 10 atomes de carbone, ou forment ensemble un reste cycloalkyle ayant de 5 à 7 atomes de carbone, portant éventuellement des ramifications alkyle, qui peut contenir un ou deux atomes d'azote, d'oxygène et/ou de soufre en tant qu'hétéroatomes, ou un reste imidazolyle, triazolyle, tétrazolyle, 3-nitro 1,2,4-triazolyle, thiazolyle, pyrrolyle, benzotriazolyle ou benzhydroxytriazolyle et

B' représente une base nucléotidique éventuellement protégée par des groupes protecteurs usuels dans la chimie des nucléotides.

4. Nucléotide protégé selon la revendication 3, caractérisé par le fait que 2 représente -CN.

5. Nucléotide protégé selon la revendication 4, caractérisé par le fait que $R^3$ représente $-CH_2-CH_2-CN$.

6. Nucléotide protégé selon la revendication 3, caractérisé par le fait que $R^2$ représente le groupe 4,4'-diméthoxy- ou 4,4',4''-triméthoxytrityle.

7. Nucléotide protégé de formule

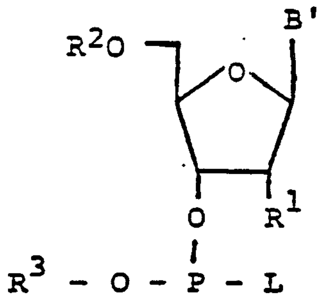

(IV)

dans laquelle

$R^1$ représente un atome d'hydrogène ou un groupe hydroxyle ou hydroxyle protégé par des groupements protecteurs usuels dans la chimie des nucléotides;

$R^2$ représente le groupe 4,4'-diméthoxy- ou 4,4',4''-triméthoxytrityle;

$R^3$ représente $-CH_2-CH_2-CN$;

B' représente une base nucléotidique éventuellement protégée par des groupes protecteurs usuels dans la chimie des nucléotides; et

L représente le groupe N,N-diméthylamino, N,N-diisopropylamino ou N-morpholino.

Fig. 1a

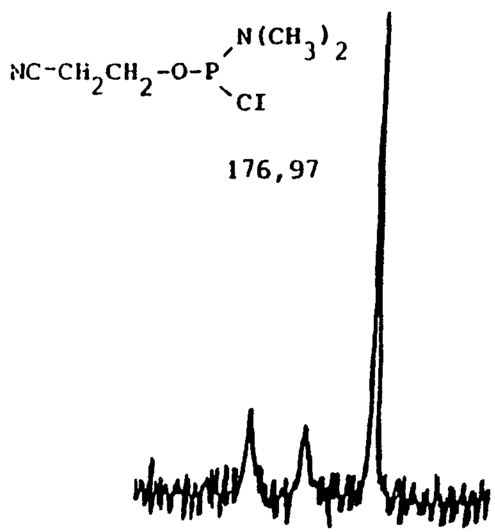

176,97

Fig. 1b

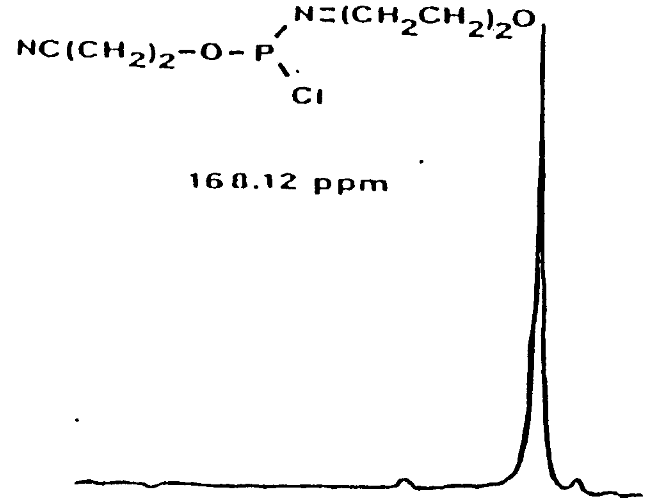

168.12 ppm

Fig. 1c

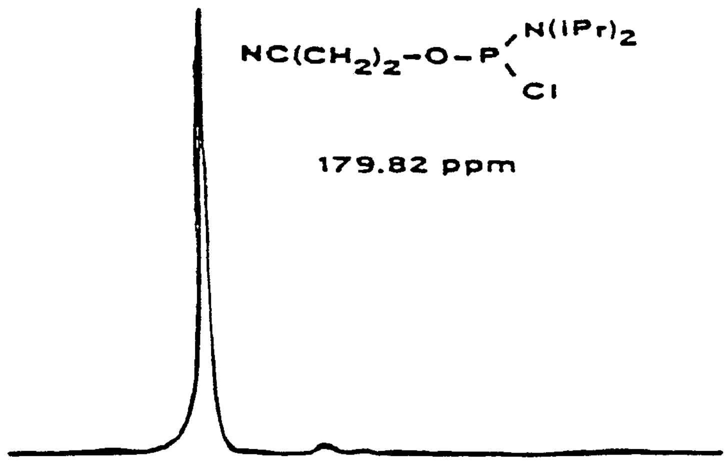


Fig. 2

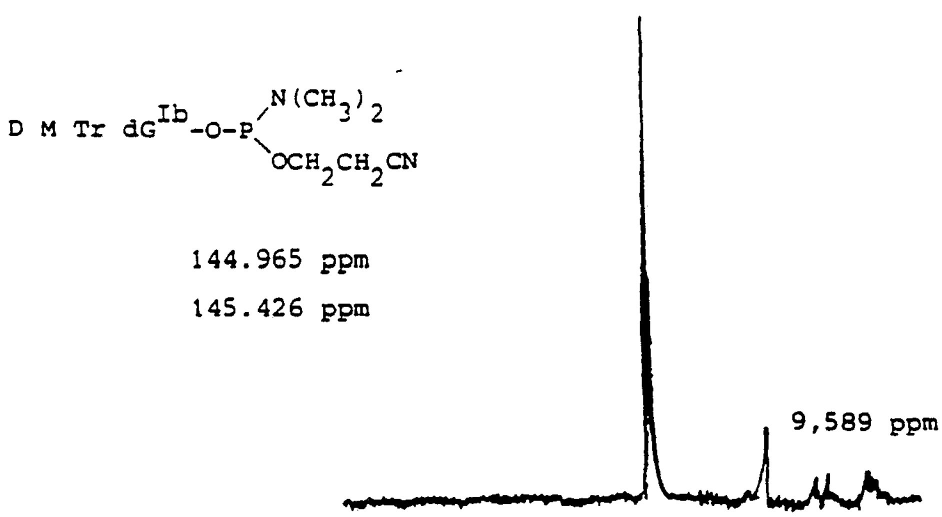

Fig. 3
HPLC des dimethoxytritylierten d (CGGTACCG)

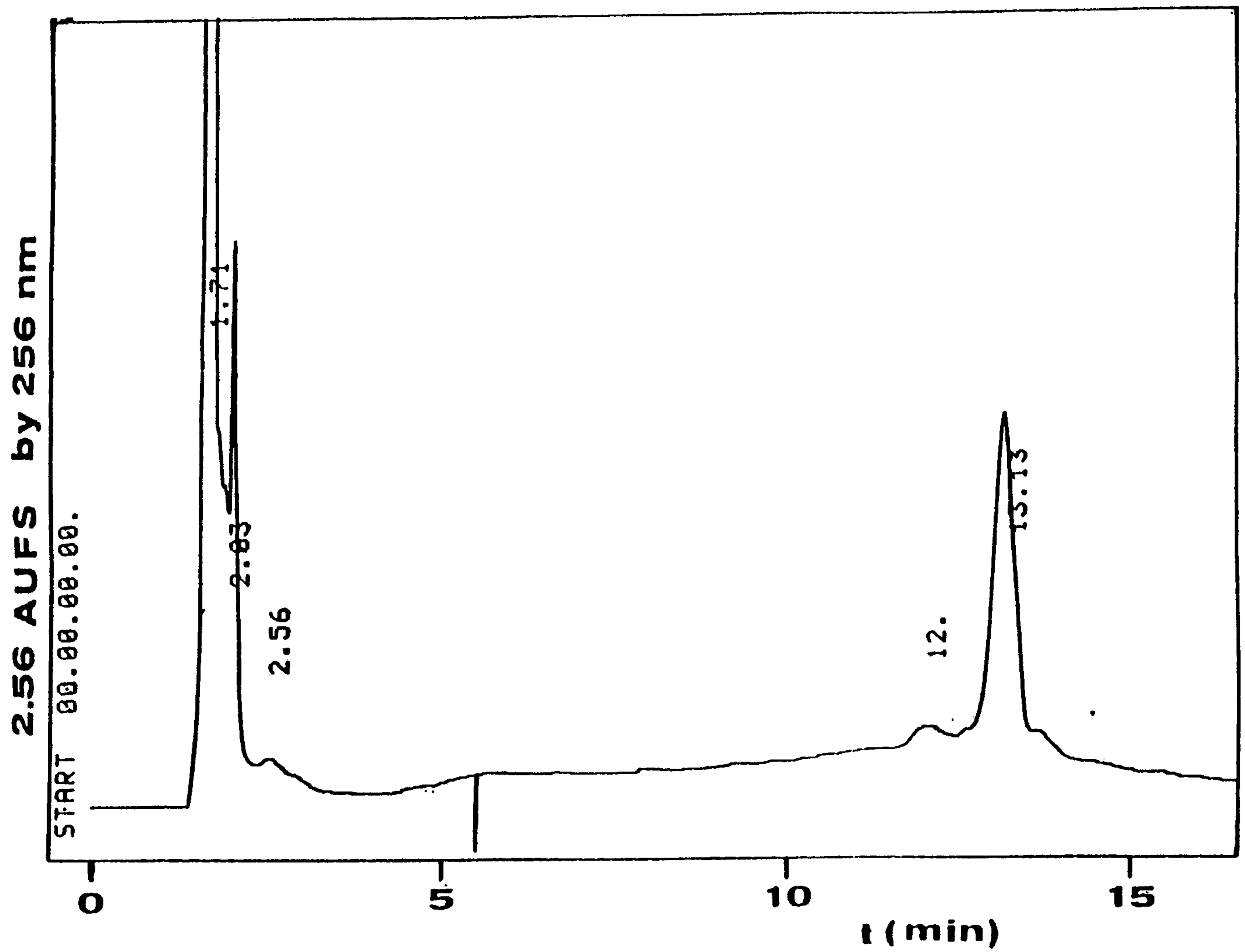

Fig. 4

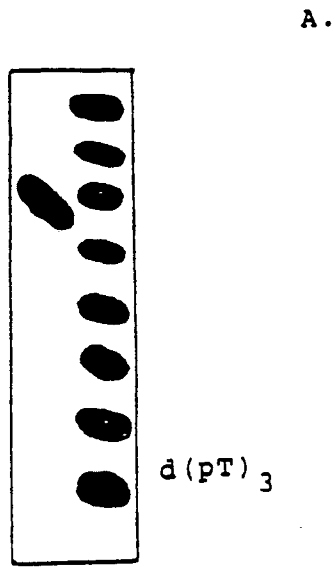


Fig. 5

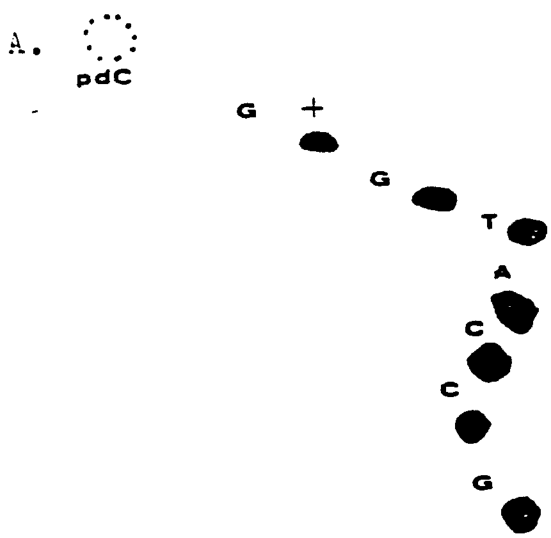

2,56 AUFS bei 256 nm

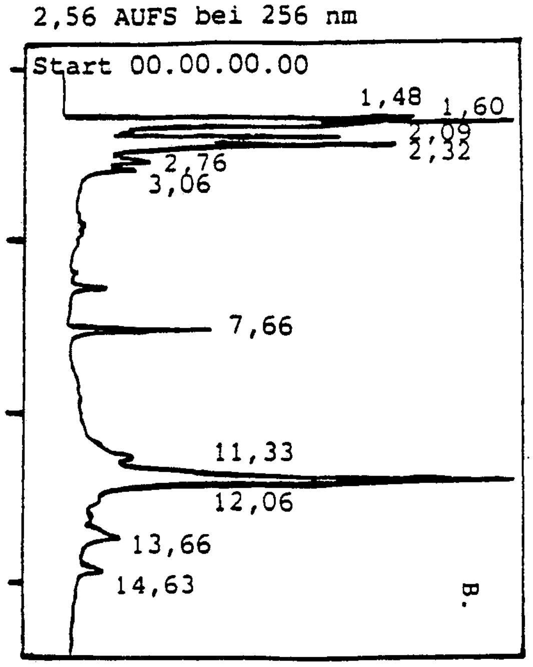


Fig. 6a

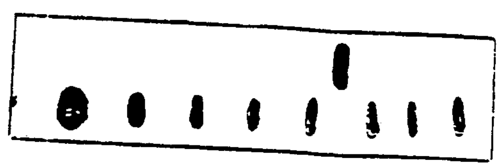

d(pT)3

B.

Fig. 6a

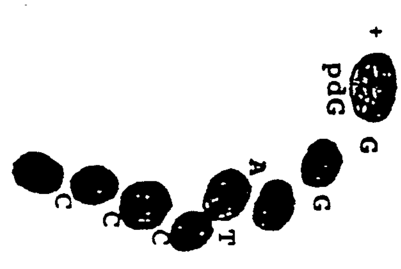


Fig. 6c

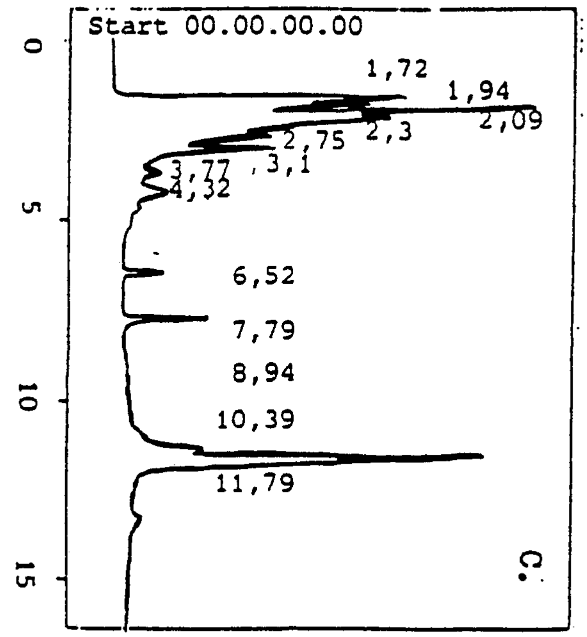


Fig. 7a

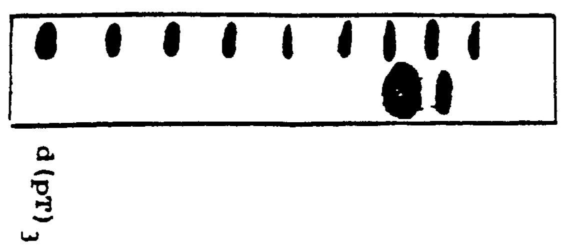


Fig. 7b

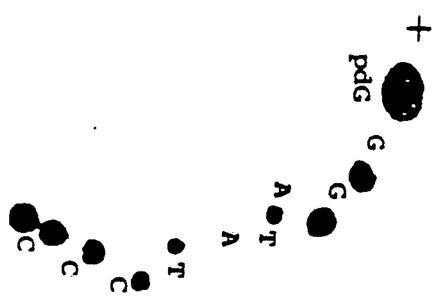


Fig. 7c